# EUROPEAN PATENT APPLICATION

(11) **EP 4 059 567 A1**
(43) Date of publication of application: **21.09.2022**
(21) Application number: 20888113.6
(22) Date of filing: 13.11.2020
(51) Int. Cl.: A61P 29/00, C08F 120/36, A61K 31/78

(54) **O-GLCNACYLATED PROTEIN-LIKE SUBSTANCE AND FIBROSIS THERAPEUTIC DRUG CONTAINING SAME**

(30) Priority: 14.11.2019 JP 2019206265
(71) Applicant: Kyushu University, National University Corporation, Nishi-ku Fukuoka-shi Fukuoka 819-0395 (JP); Somar Corporation, Tokyo 104-8109 (JP)
(72) Inventor: ISE Hirohiko, Fukuoka-shi, Fukuoka 819-0395 (JP); MATSUO Saori, Tokyo 104-8109 (JP)
(74) Representative: EP&C
(86) International application number: PCT/JP2020/042362
(87) International publication number: WO 2021/095828

(57) **Abstract**

An O-GlcNAcylated protein-like substance having an N-acetylglucosamine unit, and at least one unit selected from the group consisting of a carboxy group-containing radically polymerizable unit, a styrene unit, a polyethylenimine unit, a poly-L-lysine unit, and a biotin unit; and a fibrosis therapeutic drug containing the O-GlcNAcylated protein-like substance.

## Description

### TECHNICAL FIELD

The present invention relates to an O-GlcNAcylated protein-like substance and a fibrosis therapeutic drug containing the same.

Priority is claimed on Japanese Patent Application No. 2019-206265 filed November 14, 2019, the content of which is incorporated herein by reference.

### BACKGROUND ART

Fibroblasts in which connective tissue constituting tissues such as internal organs is abnormally proliferated or activated are accumulated in a fibrotic site to produce a large amount of collagen or other proteins, and these products adhere to the tissues causing tissue fibrosis. Fibrosis can occur in almost all tissues, and for example, liver fibrosis is a common pathological condition caused by various factors such as an autoimmunity mechanism, intrahepatic cholestasis, drug-induced reactions, metal dysbolism, and congested liver, in addition to persistent infection with hepatitis virus, excessive alcohol intake, and non-alcoholic steatohepatitis, and proceeds as activated stellate cells are transformed into myofibroblast-like cells to produce an extracellular matrix. Along with the progress of fibrosis, an extracellular matrix such as collagen is excessively deposited in the liver tissue, and as a result, hepatocyte dysfunction or portal vein hyperactivity progresses in liver cirrhosis.

It is known that tissues other than the liver, for example, organs such as the kidney, the lung, the heart, and the pancreas are also susceptible to various diseases due to fibrosis caused by myofibroblasts. Since such fibrotic states of various organs progress to further diseases in this manner, several improvement methods have been proposed, which are expected to lead to effective treatment of liver cirrhosis or liver cancer, and other diseases related to fibrosis of organs by preventing or ameliorating fibrosis.

For example, Patent Document 1 proposes a prophylactic or therapeutic agent for a tissue fibrotic disease, including a metal chelate compound or a pharmaceutically acceptable salt thereof as an active ingredient for a prophylactic or therapeutic agent for a tissue fibrotic disease.

In addition, Patent Document 2 proposes a fibrosis inhibitor containing, as an active ingredient, a polynucleotide encoding a protein belonging to a Sema6 family or a partial fragment thereof; a protein belonging to the Sema6 family or a partial fragment thereof; or an agonist for a receptor in which ligand is the protein or a partial fragment thereof in order to prevent the onset of fibrosis, delay the progress of fibrosis, or ameliorate fibrosis.

However, many antifibrotic substances have been identified and reported from tests using cultured cells and animal experiments, but specific and effective therapeutic drugs for liver fibrosis have not yet been proposed in clinical practice.

In addition, it has recently been found that the blood concentration of a growth and differentiation factor 15 (hereinafter simply referred to as GDF15) is increased in patients with various malignant tumors. GDF15 is a cytokine exhibiting various biological characteristics belonging to a TGF-β superfamily, and is remarkably expressed in the placenta, the kidney, the intestine, the prostate, the choroid plexus, or the like. In addition, GDF15 induces gene expression in response to stress such as hypoxia, inflammation, UV exposure, and tissue damage, in addition to those tissues or cells (Non-Patent Document 1). Furthermore, GDF15 is also known to be involved in proliferation, metastasis of cancer cells, treatment resistance, and the like, in addition to anti-inflammatory, cardioplegic, neuroprotective, and appetite or metabolism-regulating actions (Non-Patent Document 2). For example, it has been reported that mesenchymal stem cells differentiated from iPS cells ameliorate doxorubicin-induced cardiomyopathy by secretion of GDF15 (Non-Patent Document 3). In addition, it has been reported that in model mice with alcohol- and carbon tetrachloride-induced hepatopathy, for example, GDF15 deficiency aggravates these pathological conditions but can ameliorate the conditions by administration of GDF15 (Non-Patent Document 4); GDF15 ameliorates non-alcoholic steatohepatitis (hereinafter referred to as NASH) (Non-Patent Document 5); in transgenic mice highly expressing GDF15, the progression of rheumatoid arthritis is suppressed (Non-Patent Document 6); and in pancreatic cancer, GDF15 is secreted by activation of NF-κB and allowed to act on the surrounding macrophages and suppress NF-κB of the macrophages, thereby stopping the production of TNFα, and the pancreatic cancer itself proliferates by avoiding attack of the macrophages by the suppression of TNFα secretion of the macrophages (Non-Patent Document 7).

As described above, it is known that GDF15 is present in an increased amount in the blood with myocardial infarction, fibrosis, or autoimmune diseases, and has an injury-protecting action through an anti-inflammatory action on pathological conditions.

### Citation List

### Patent Documents

[Patent Document 1]
   Japanese Unexamined Patent Application, First Publication No. 2011-190257
[Patent Document 2]
   Japanese Unexamined Patent Application, First Publication No. 2018-21026

### Non-Patent Documents

[Non-Patent Document 1]
   Unsicker K, Spittau B, Krieglstein K: The multiple facets of the TGF-beta family cytokine growth/differentiation factor-15/macrophage inhibitory cytokine-1. Cytokine Growth Factor Rev 2013, 24(4): 373-384.
[Non-Patent Document 2]
   Wang X, Baek SJ, Eling TE: The diverse roles of nonsteroidal anti-inflammatory drug activated gene (NAG-1/GDF15) in cancer. Biochem Pharmacol 2013, 85(5): 597-606.
[Non-Patent Document 3]
   Zhang Y, Liang X, Liao S, Wang W, Wang J, Li X, Ding Y, Liang Y, Gao F, Yang M, Fu Q, Xu A, Chai YH, He J, Tse HF, Lian Q. Potent Paracrine Effects of human induced Pluripotent Stem Cell-derived Mesenchymal Stem Cells Attenuate Doxorubicin-induced Cardiomyopathy. Sci Rep. 2015 Jun 9; 5: 11235. doi: 10.1038/srep11235
[Non-Patent Document 4]
   Chung HK, Kim JT, Kim HW, Kwon M, Kim SY, Shong M, Kim KS, Yi HS. GDF15 deficiency exacerbates chronic alcohol- and carbon tetrachloride-induced liver injury. Sci Rep. 2017 Dec 8; 7(1): 17238. doi: 10.1038/s41598-017-17574-w.
[Non-Patent Document 5]
   Kim KH, Kim SH, Han DH, Jo YS, Lee YH, Lee MS. Growth differentiation factor 15 ameliorates nonalcoholic steatohepatitis and related metabolic disorders in mice. Sci Rep. 2018 May 1; 8(1): 6789. doi: 10.1038/s41598-018-25098-0.
[Non-Patent Document 6]
   Breit SN, Johnen H, Cook AD, Tsai VW, Mohammad MG, Kuffner T, Zhang HP, Marquis CP, Jiang L, Lockwood G, Lee-Ng M, Husaini Y, Wu L, Hamilton JA, Brown DA. The TGF-β superfamily cytokine, MIC-1/GDF15: a pleotrophic cytokine with roles in inflammation, cancer and metabolism. Growth Factors. 2011 Oct; 29(5): 187-95. doi: 10.3109/08977194.2011.607137. Epub 2011 Aug 11.
[Non-Patent Document 7]
   Ratnam NM, Peterson JM, Talbert EE, Ladner KJ, Rajasekera PV, Schmidt CR, Dillhoff ME, Swanson BJ, Haverick E, Kladney RD, Williams TM, Leone GW, Wang DJ, Guttridge DC. NF-κB regulates GDF-15 to suppress macrophage surveillance during early tumor development. J Clin Invest. 2017 Oct 2; 127 (10): 3796-3809. doi: 10.1172/JCI91561. Epub 2017 Sep 11.

### DISCLOSURE OF INVENTION

### Technical Problem

Despite the findings described above, an effective prophylactic or therapeutic agent for tissue fibrosis has not yet been developed, and although GDF15 is known to have an injury-protecting action through an anti-inflammatory action on pathological conditions, there is currently no method for increasing GDF15 expression in a diseased area or the vicinity thereof to effectively obtain an anti-inflammatory action or proliferation suppression.

Therefore, an object of the present invention is to provide an O-GlcNAcylated protein-like substance that contributes to an increase in GDF15 expression, and a fibrosis therapeutic drug containing the same.

### Solution to Problem

From the fact that an O-GlcNAcylated protein released upon cell injury binds to vimentin on the surface of another cell, the present inventors have found a polymer that functions in the same manner as the O-GlcNAcylated protein, and also found that it is possible to effectively obtain an anti-inflammatory action or proliferation suppression due to an increase in GDF15 expression by using the polymer so that the polymer effectively binds to injured tissues or cells, thereby leading to completion of the present invention.

That is, the present invention includes [1] to [5] below, which relate to an O-GlcNAcylated protein-like substance and a fibrosis therapeutic drug containing the same.
[1] An O-GlcNAcylated protein-like substance having an N-acetylglucosamine unit and at least one unit selected from the group consisting of a carboxy group-containing radically polymerizable unit, a styrene unit, a polyethylenimine unit, a poly-L-lysine unit, and a biotin unit.
[2] The O-GlcNAcylated protein-like substance described in [1],
   in which the carboxy group-containing radically polymerizable unit is at least one selected from the group consisting of acrylic acid, methacrylic acid, itaconic acid, maleic acid, crotonic acid, 2-carboxyethyl acrylate, and 2-carboxyethyl methacrylate.
[3] The O-GlcNAcylated protein-like substance described in [1] or [2],
   in which the O-GlcNAcylated protein-like substance has no alkyl chain.
[4] The O-GlcNAcylated protein-like substance described in any one of [1] to [3],
   in which the O-GlcNAcylated protein-like substance is a compound represented by Chemical Formula (1) or (2). 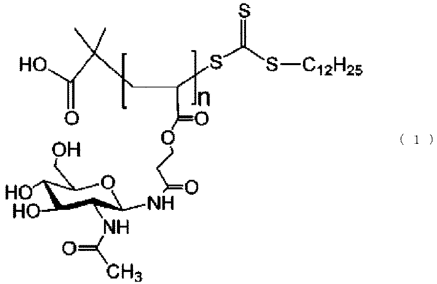 In Formula (1), n is an integer of 3 to 100. 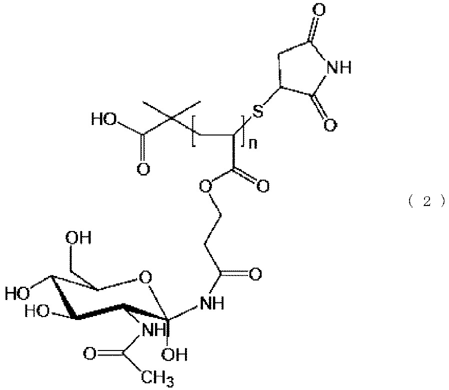 In Formula (2), n is an integer of 3 to 100.
[5] A fibrosis therapeutic drug containing the O-GlcNAcylated protein-like substance described in any one of [1] to [4] as an active ingredient.

### Advantageous Effects of Invention

According to the present invention, it is possible to provide an O-GlcNAcylated protein-like substance and a fibrosis therapeutic drug containing the same.

### BRIEF DESCRIPTION OF DRAWINGS

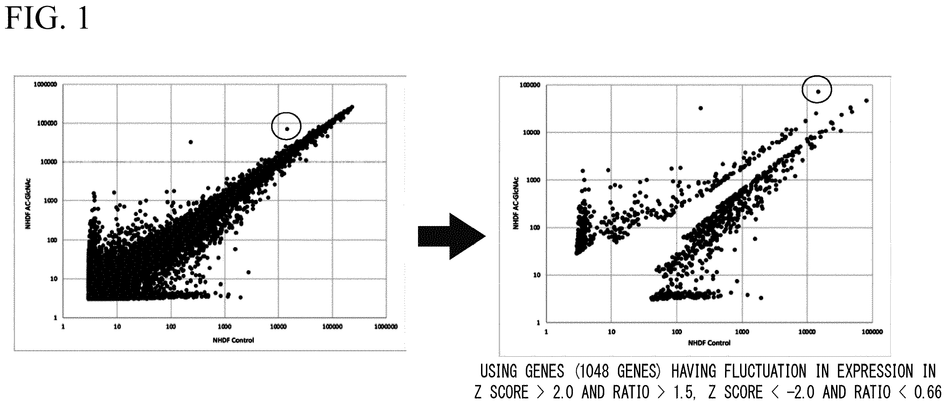

Fig. 1 is a graph showing the results of investigating an effect of AC-GlcNAc (10-mer) on human fibroblasts (hereinafter also referred to as NHDF) with a DNA microarray using AC-GlcNAc as an O-GlcNAcylated protein-like substance.

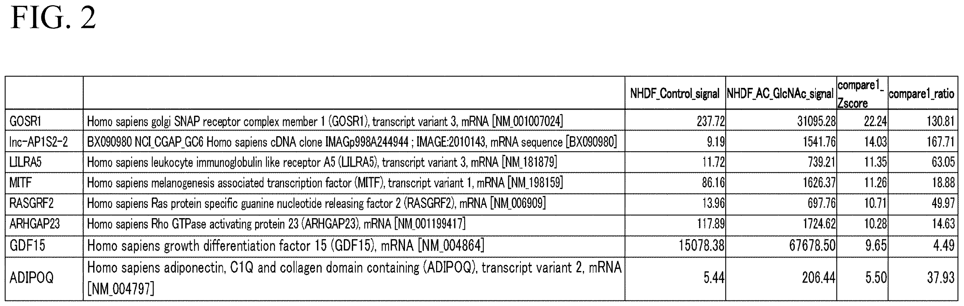

Fig. 2 shows the results of the microarray shown in Fig. 1 for each protein.

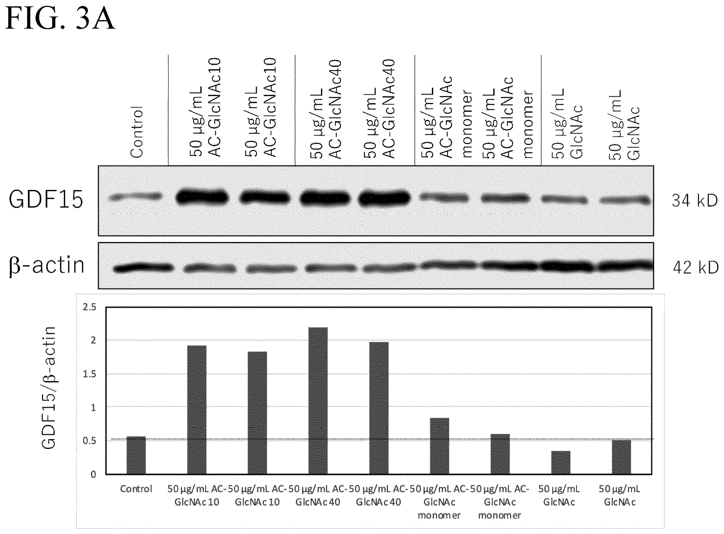

In Fig. 3A, the upper figure shows a Western blot showing an increase in the expression of GDF15 at the protein level, and the expression of β-actin after AC-GlcNAc (10-mer), AC-GlcNAc (40-mer), AC-GlcNAc (monomer), or GlcNAc as O-GlcNAcylated protein-like substances was added to NHDF, and culture was performed for 24 hours. Furthermore, the control is one to which the O-GlcNAcylated protein-like substance was not added.
   In addition, the lower figure is a graph showing ratios of GDF15 to β-actin (GDF15/β-actin).

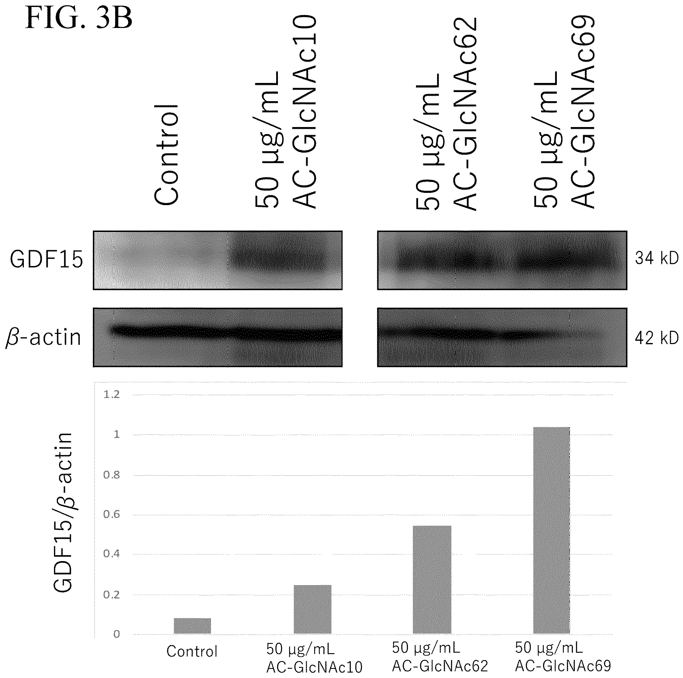

In Fig. 3B, the upper figure shows a Western blot showing an increase in the expression of GDF15 at the protein level, and the expression of β-actin after AC-GlcNAc (10-mer), AC-GlcNAc (62-mer), or AC-GlcNAc (69-mer) as O-GlcNAcylated protein-like substances was added to NHDF, and culture was performed for 24 hours. Furthermore, the control is one to which the O-GlcNAcylated protein-like substance was not added.
   In addition, the lower figure is a graph showing ratios of GDF15 to β-actin (GDF15/β-actin).

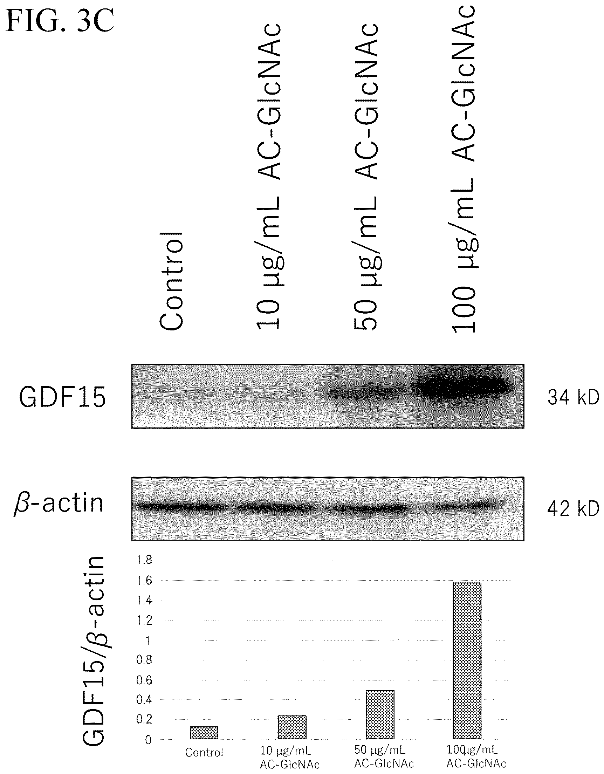

In Fig. 3C, the upper figure shows a Western blot showing an increase in the expression of GDF15 at the protein level, and the expression of β-actin after 10 µg/mL, 50 µg/mL, or 100 µg/mL of AC-GlcNAc (10-mer) was added as O-GlcNAcylated protein-like substances to NHDF, and culture was performed for 24 hours.
   In addition, the lower figure is a graph showing ratios of GDF15 to β-actin (GDF15/β-actin).

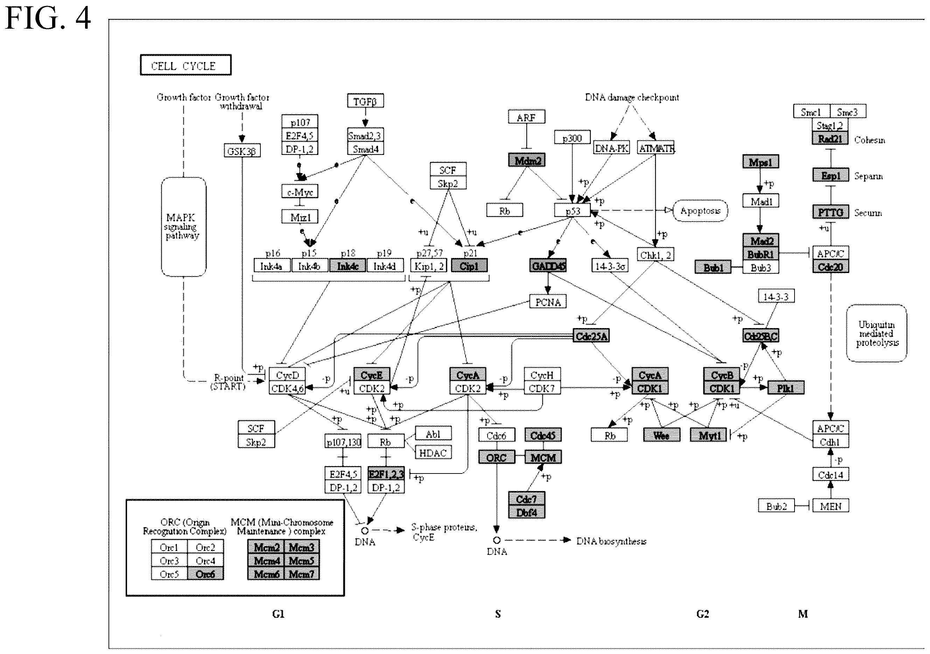

Fig. 4 shows the results of analyzing pathways of differentially expressed genes from the results of the microarray shown in Fig. 1.

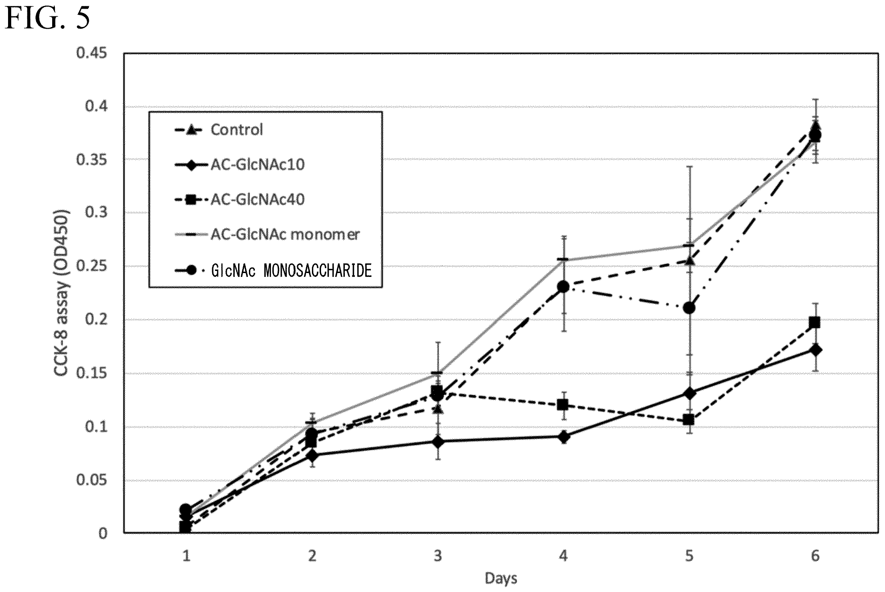

Fig. 5 shows the proliferation rates of NHDF in a case where 50 mL of an O-GlcNAcylated protein-like substance [AC-GlcNAc (10-mer) or AC-GlcNAc (40-mer)], or an AC-GlcNAc monomer or N-acetylglucosamine as other components was added to NHDF, and culture was performed for 6 days. The control is one to which the O-GlcNAcylated protein-like substance was not added.

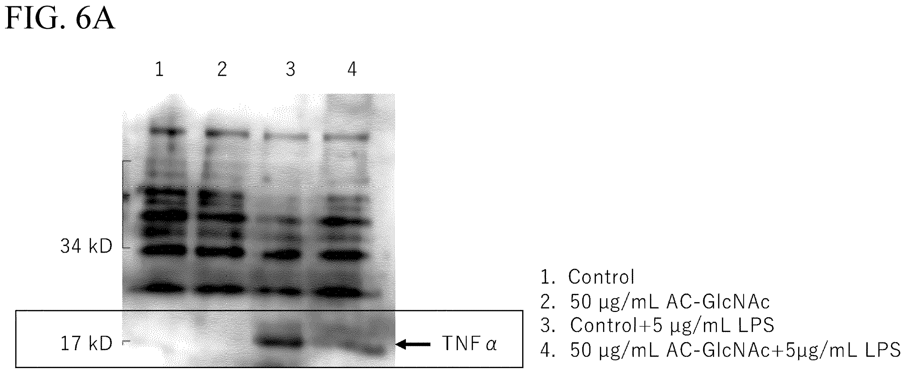

Fig. 6A shows the results of Western blotting detection of intracellular production of TNFα using AC-GlcNAc (10-mer) as an O-GlcNAcylated protein-like substance, in which 50 µg/mL of AC-GlcNAc (10-mer) was added to NHDF, 24 hours later, 5 µg/mL of a lipopolysaccharide (hereinafter also referred to as LPS) was added thereto, and detection was then performed 8 hours after the addition.

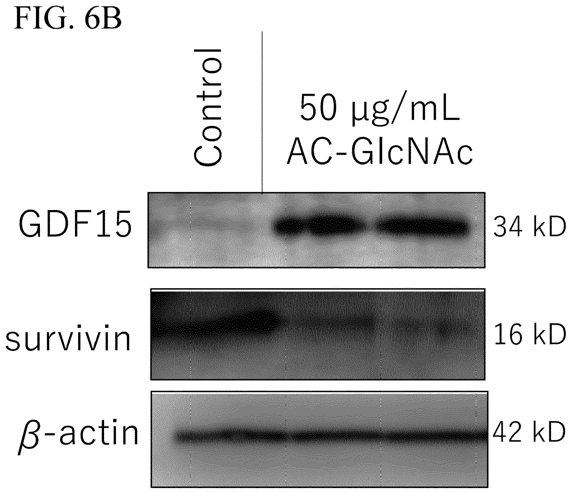

Fig. 6B shows the results of Western blotting detection of intracellular production of GDF15, survivin, and β-actin using AC-GlcNAc (10-mer) as an O-GlcNAcylated protein-like substance, in which 50 µg/mL of AC-GlcNAc (10-mer) was added to NHDF, and detection was then performed 24 hours after the addition.

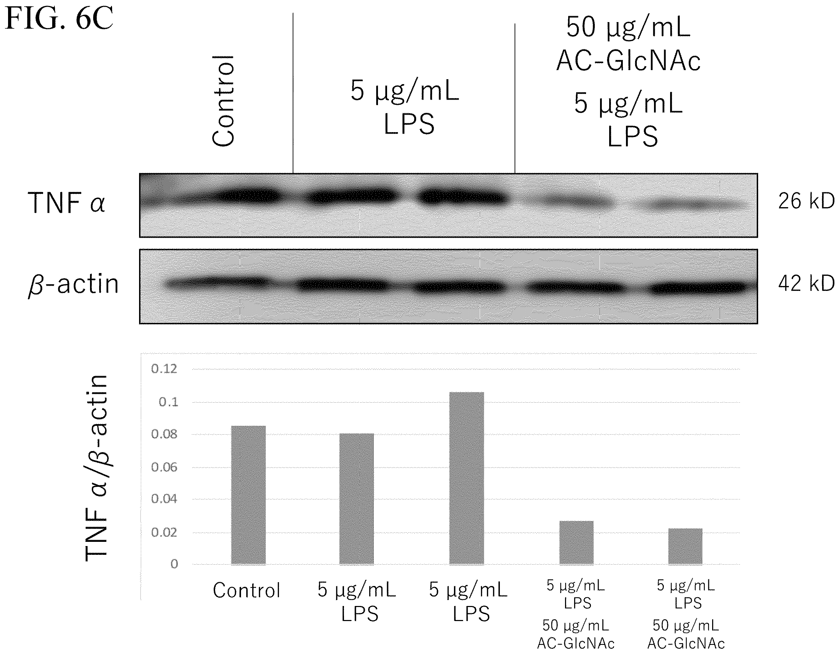

Fig. 6C is a graph showing the results of values of intracellular ratios of TNFα to β-actin (TNFα/β-actin) using AC-GlcNAc (10-mer) as an O-GlcNAcylated protein-like substance, in which 50 µg/mL of AC-GlcNAc (10-mer) was added to NHDF, 24 hours later, 5 µg/mL of LPS was added thereto, and the values of intracellular ratios were obtained 8 hours after the addition.

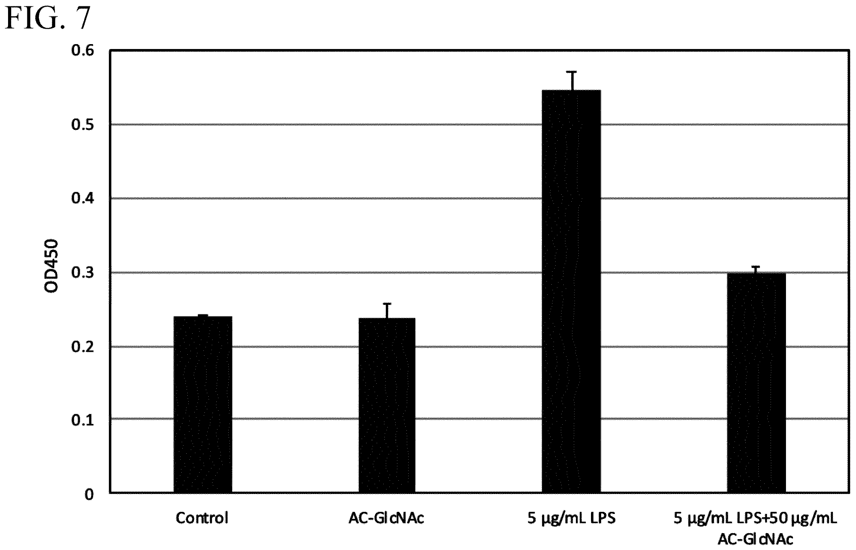

Fig. 7 shows the results of ELISA detection of amounts of TNFα secreted in a medium using AC-GlcNAc (10-mer) as an O-GlcNAcylated protein-like substance, in which 50 µg/mL of AC-GlcNAc (10-mer) was added to NHDF, 24 hours later, 5 µg/mL of LPS was added thereto, and detection was then performed 24 hours after the addition.

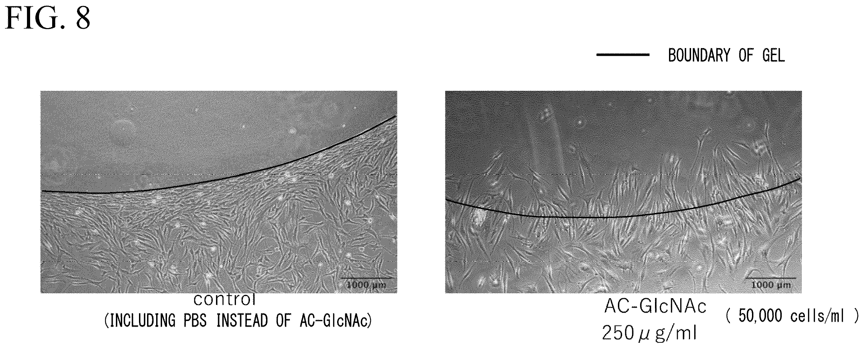

Fig. 8 shows the results of observing the state of NHDF with a phase-contrast microscope in a case where a glass plate was placed in a petri dish, an agarose gel mixed with AC-GlcNAc (10-mer) was added dropwise thereto, and NHDF was further seeded on the entire glass plate, followed by performing incubation for one day; and observing the invasion state into cells on the bottom surface of the agarose gel. The control shows the results of using PBS instead of AC-GlcNAc (10-mer).

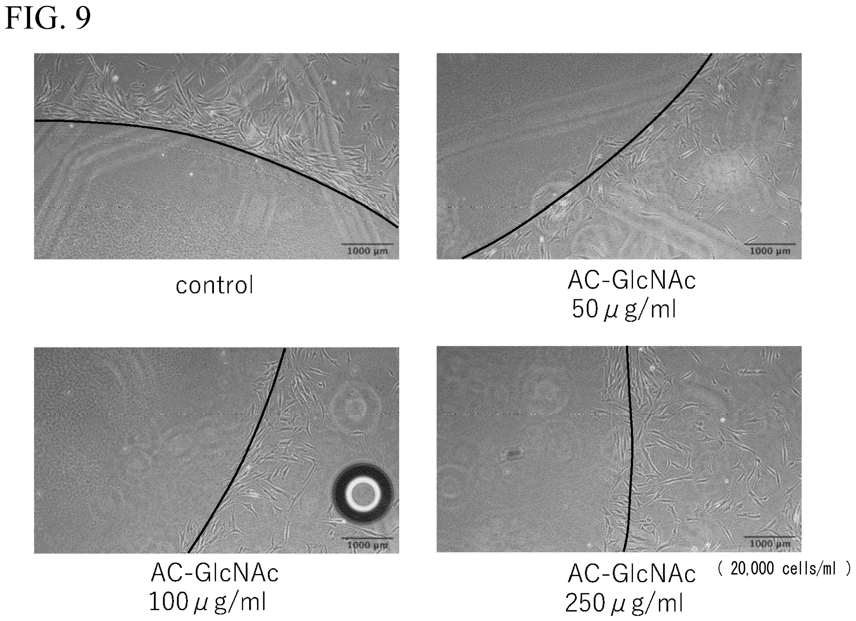

Fig. 9 shows the results of observing the state of NHDF with a phase-contrast microscope in a case where the blending amounts of the O-GlcNAcylated protein-like substance into the agarose gel were 50 µg/mL, 100 µg/mL, or 250 µg/mL; and observing the invasion state into cells on the bottom surface of the agarose gel.

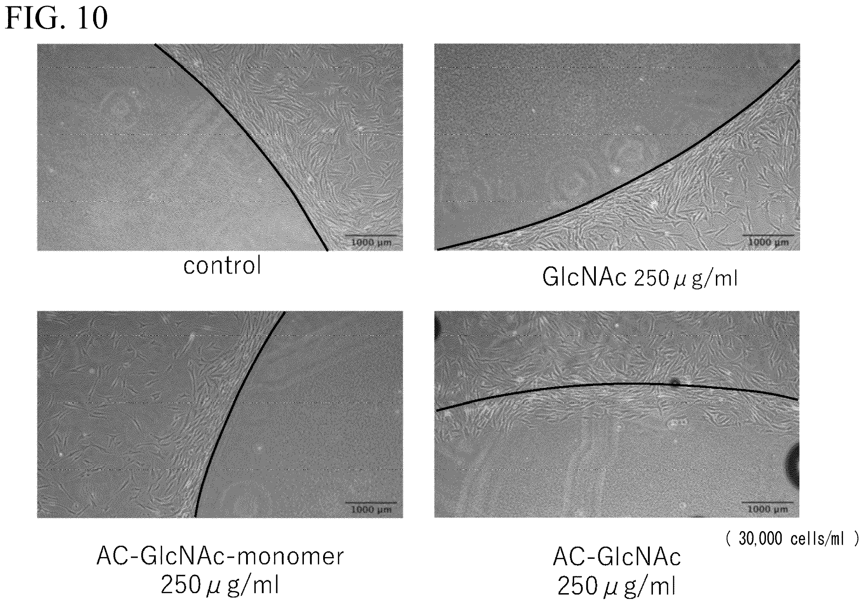

Fig. 10 shows the results of observing the state of NHDF with a phase-contrast microscope in a case where an O-GlcNAcylated protein-like substance prepared in Polymerization Example 1, an AC-GlcNAc monomer prepared in Polymerization Example 5, or N-acetylglucosamine (GlcNAc) used in Example 2 or PBS was used; and observing the invasion state into cells on the bottom surface of the agarose gel.

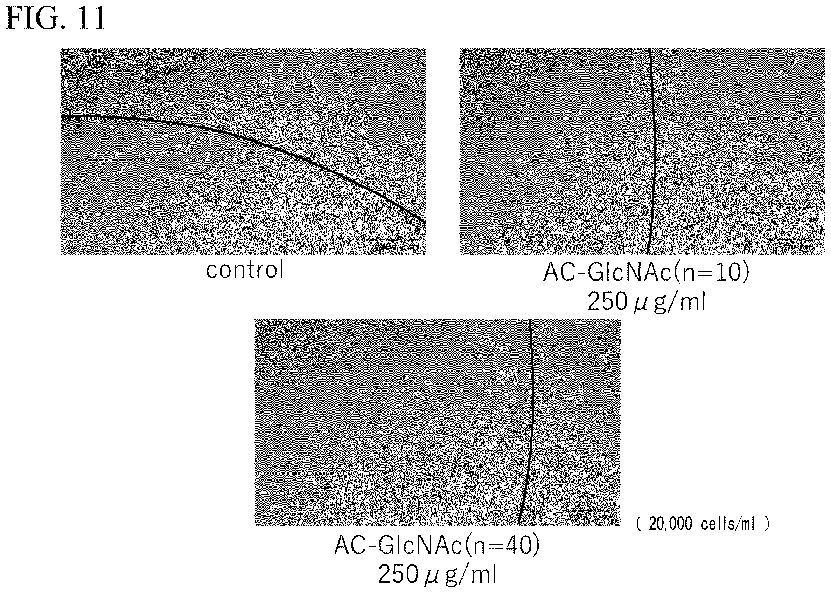

Fig. 11 shows the results of observing the state of NHDF with a phase-contrast microscope in a case where an O-GlcNAcylated protein-like substance prepared in Polymerization Example 1 or an O-GlcNAcylated protein-like substance prepared in Polymerization Example 2 was used as an O-GlcNAcylated protein-like substance; and observing the invasion state into cells on the bottom surface of the agarose gel.

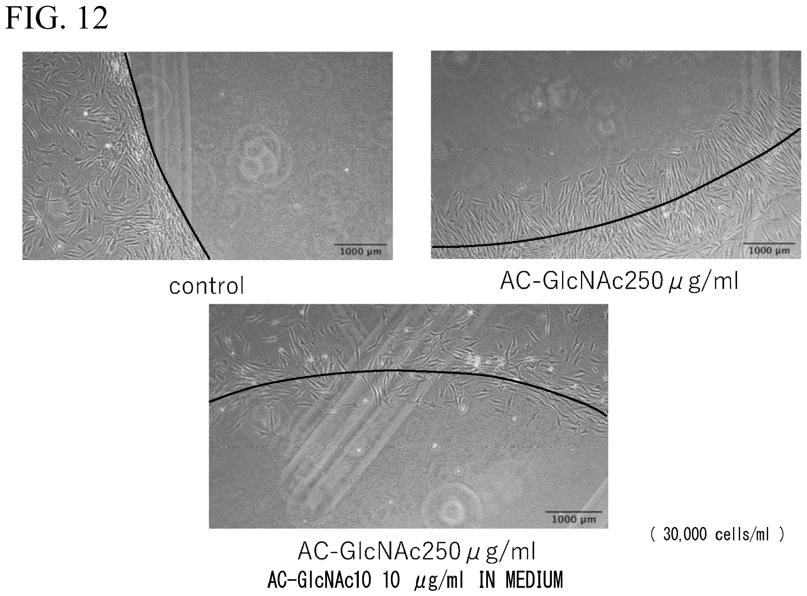

Fig. 12 shows the results of evaluating the chemotaxis of NHDF by incorporating 10 µg/mL of an O-GlcNAcylated protein-like substance of Polymerization Example 1 in a medium using a petri dish in which a gel containing an O-GlcNAcylated protein-like substance of Polymerization Example 1 was added dropwise to an agarose gel.

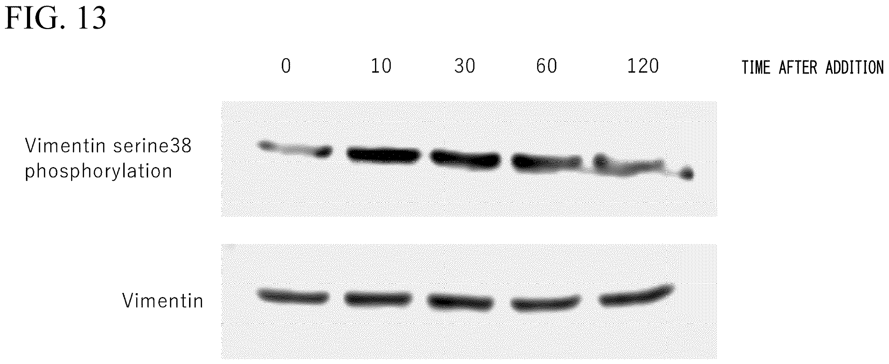

Fig. 13 shows the results of Western blotting detection of the phosphorylation of vimentin using anti-phosphorylated vimentin serine-38 antibody at 10 minutes, 30 minutes, 60 minutes, and 120 minutes after the addition of 50 µg/mL of an O-GlcNAcylated protein-like substance to NHDF.

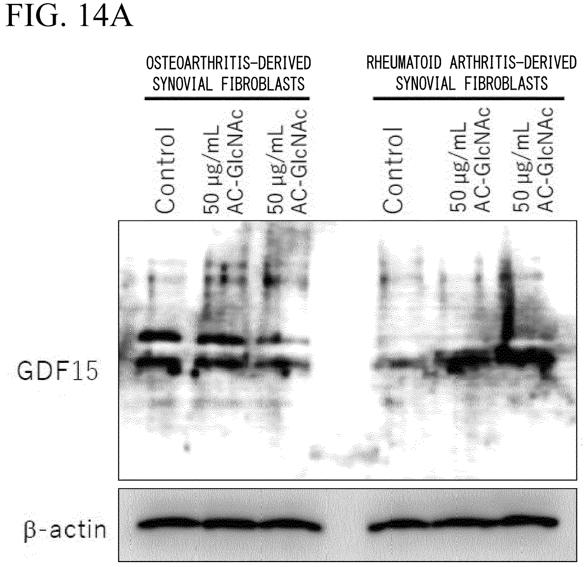

Fig. 14A shows the results of Western blotting detection of intracellular production of GDF15 and β-actin using AC-GlcNAc (10-mer) as an O-GlcNAcylated protein-like substance, in which 50 µg/mL of AC-GlcNAc (10-mer) was added to osteoarthritis-derived synovial fibroblasts and rheumatoid arthritis-derived synovial fibroblasts, and culture was performed for 24 hours.

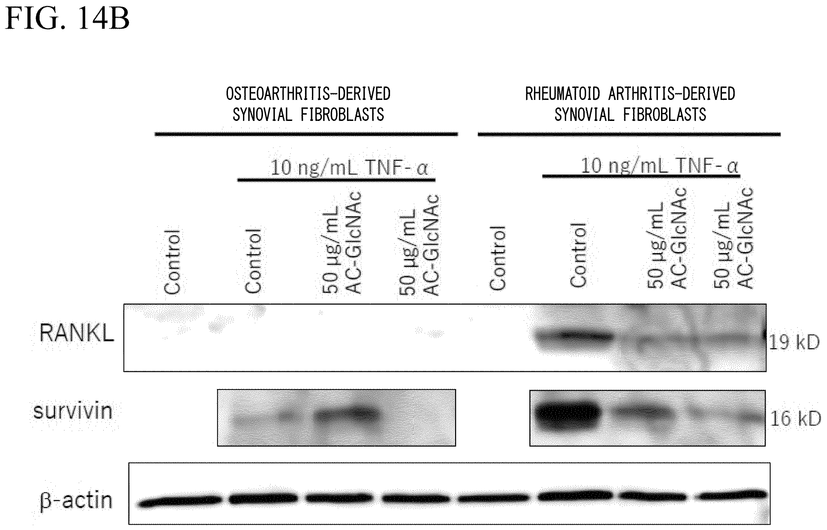

Fig. 14B shows the results of Western blotting detection of the expression of a receptor activator of an NF-κB ligand (hereinafter referred to as RANKL), survivin, and β-actin at 6 hours after the addition of 10 ng/mL of TNFα to osteoarthritis-derived synovial fibroblasts and rheumatoid arthritis-derived synovial fibroblasts using AC-GlcNAc (10-mer) as an O-GlcNAcylated protein-like substance.

### BEST MODE FOR CARRYING OUT THE INVENTION

An O-GlcNAcylated protein is one post-translational modification in which O-GlcNAc is bound to a hydroxyl group of serine and threonine residues of a protein, and is a protein formed by O-GlcNAcylation of an intracellular protein and represented by, for example, the following general formula. This O-GlcNAcylated protein is expected to leak from an injured cell and act in a similar manner to an alamine molecule. That is, 4,000 or more kinds of proteins such as a nuclear protein and a cytoskeleton are O-GlcNAcylated, and the O-GlcNAcylated protein is constantly present in a large amount in cells. In a case where damage, for example, fibrosis occurs in a cell, this O-GlcNAcylated protein is released in a large amount to the outside of the cell, and binds to and acts on vimentin on the cell surface of another cell to influence intracellular, differentially expressed genes and to express GDF15.

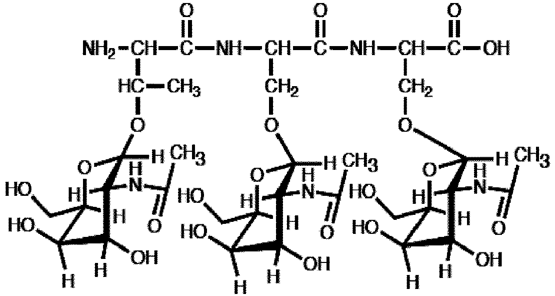

The O-GlcNAcylated protein-like substance of the present invention is a compound having the same function as the above-mentioned O-GlcNAcylated protein and includes a specific N-acetylglucosamine group-containing polymer. The fibrosis therapeutic drug contains this O-GlcNAcylated protein-like substance, and can ameliorate fibrosis by binding to and acting on an affected area such as diseased tissues and cells.

Hereinafter, the O-GlcNAcylated protein-like substance and the fibrosis therapeutic drug containing the same of the present invention will be described in detail.

### [O-GlcNAcylated Protein-Like Substance]

In the present invention, the O-GlcNAcylated protein-like substance is a polymer having an N-acetylglucosamine unit and at least one unit selected from the group consisting of a carboxy group-containing radically polymerizable unit, a styrene unit, a polyethylenimine unit, a poly-L-lysine unit, and a biotin unit. These N-acetylglucosamine group-containing polymers preferably have a mass-average molecular weight in the range of 3,000 to 30,000 and a number-average molecular weight in the range of 2,500 to 25,000, and the N-acetylglucosamine group-containing polymers more preferably have a mass-average molecular weight in the range of 3,000 or greater and 30,000 or less, or a number-average molecular weight of 2,500 or greater and 25,000 or less since their actions on cells are improved.

For the N-acetylglucosamine group-containing polymer used as an O-GlcNAcylated protein-like substance, the mass-average molecular weight is preferably in the range of 4,000 to 20,000 and the number-average molecular weight is preferably in the range of 3,000 to 10,000 from the viewpoint of the yield of the compound by polymerization; and the mass-average molecular weight is preferably in the range of 4,000 to 15,000 and the number-average molecular weight is preferably in the range of 3,000 to 8,000 from the viewpoint of the expression of GDF15.

In addition, a ratio (Mw/Mn) of the mass-average molecular weight (Mw) to the number-average molecular weight (Mn) is preferably in the range of 1.0 to 2.5; and more preferably in the range of 1.2 to 2.2 from the viewpoint of the expression of GDF15 and suppression of the expression of RANKL and survivin.

Examples of the N-acetylglucosamine unit include N-acetylglucosamine, chitobiose, chitotriose, chitotetraose, chitopentaose, and chitohexaose. Examples of the N-acetylglucosamine sugar chain group-containing organic resin compound include a compound having, as the N-acetylglucosamine sugar chain group, an N-acetylglucosamine group or a chitopolyose group in which 2 to 6 N-acetylglucosamine groups are bound to each other.

In addition, examples of the carboxy group-containing radically polymerizable unit include acrylic acid, methacrylic acid, itaconic acid, maleic acid, crotonic acid, 2-carboxyethyl acrylate, and 2-carboxyethyl methacrylate.

A method for producing the N-acetylglucosamine group-containing polymer is not particularly limited, and radical polymerization, living radical polymerization, a reversible addition-cleaving chain transfer polymerization method (RAFT polymerization method) using a chain transfer agent (RAFT agent), or the like can be used. Examples of a method for producing a polymer obtained by polymerizing monomers having an N-acetylglucosamine group include a method of polymerizing monomers in which a compound having a hydrophobic group such as 2-carboxyethyl acrylate is bound to a reducing terminal of an N-acetylglucosamine. More detailed examples of the production method include a method in which acrylic monomers having an N-acetylglucosamine group introduced therein are obtained by subjecting 2-carboxyethyl acrylate, which is a compound having a hydrophobic group, and an aminated N-acetylglucosamine to a condensation reaction by DMT-MM, and then the monomers are polymerized to produce the polymer. In addition, examples of a method for producing a polymer having N-acetylglucosamine bound to the polymer compound include a method in which a polymer compound having a hydrophobic group such as polyethylenimine and poly-L-lysine, which is a cationic polymer, is bound at a reducing terminal of an N-acetylglucosamine sugar chain.

Here, the method for binding the N-acetylglucosamine sugar chain to the compound is not particularly limited, and for example, an amino group of a compound having the amino group and a reducing terminal of an N-acetylglucosamine sugar chain can be bound to each other by a reduction amination method. In addition, a hydroxy group of the N-acetylglucosamine sugar chain may be substituted with a carboxy group, and bound to an amino group of a compound having the amino group by a 1-ethyl-3-(3-dimethylaminopropyl)carbodiimide (EDC) coupling method or the like.

In addition, it is preferable to use the RAFT polymerization method to control a molecular weight since the molecular weight distribution can be narrowed. In the control of the molecular weight, a ratio (Mw/Mn) of the weight-average molecular weight (Mw) to the number-average molecular weight (Mn) is preferably set to 1.0 or greater and less than 1.5 since a large amount of N-acetylglucosamine can be present on the surface of a cell culture material and more mesenchymal stem cells in a cell population can be captured.

By way of a specific example of the polymerization method, N-acetylglucosamine and 2-carboxyethyl acrylate as the compound having a hydrophobic group are mixed at a molar ratio of 1:1 and subjected to a condensation reaction in a solution such as DMT-MM, DMSO, and water to prepare an AC-GlcNAc monomer. Poly(N-ethylacrylic acid-O-2-acetamide-2-deoxy-β-D-glucopyranosylamine) (hereinafter referred to as an AC-GlcNAc polymer) can be obtained by the RAFT polymerization method. In addition, it is preferable to remove an alkyl chain derived from the RAFT polymerization method in order to improve the activity of the O-GlcNAcylated protein-like substance of the present invention with cells. Examples of a method for removing the alkyl chain include a method in which a terminal of a polymer obtained by the RAFT polymerization method is thiolated with a reducing agent or the like, and then reacted with maleimide.

In addition, the O-GlcNAcylated protein-like substance of the present invention preferably includes 3 to 100 N-acetylglucosamine groups per molecule. In a case where the number of N-acetylglucosamine groups present is less than that above, the binding property with vimentin tends to be reduced, whereas in a case where the number of N-acetylglucosamine groups present is more than that above, an amount of N-acetylglucosamine per unit area decreases, whereby the binding property with vimentin tends to be reduced. Therefore, the number is more preferably in the range of 8 to 80, still more preferably in the range of 10 to 70, particularly preferably in the range of 10 to 69, and most preferably in the range of 10 to 50.

### <Fibrosis Therapeutic Drug>

The fibrosis therapeutic drug of the present invention contains the above-mentioned O-GlcNAcylated protein-like substance, and can be administered into humans or other mammals (for example, mice, rats, hamsters, rabbits, cats, dogs, cows, sheep, and monkeys) orally or parenterally (by intravenous administration, subcutaneous administration, transdermal administration, transpulmonary administration, transmucosal administration, rectal administration, or the like). The fibrosis therapeutic drug of the present invention can be prepared by mixing the O-GlcNAcylated protein-like substance with a pharmaceutically acceptable carrier (an excipient, a binder, a disintegrator, a disintegration aid, a lubricant, a wetting agent, or the like) and additive, and the like commonly used for oral or parenteral administration, and formulating into a desired form such as a granule, a powder, a tablet (including a sugar-coated tablet), a pill, a buccal agent, a capsule, a syrup, a liquid, an emulsion, a suspension, a cream, an ointment, an eye drop, an injection, a drip, a nasal drop, a patch, and a suppository. The content of the O-GlcNAcylated protein-like substance in the fibrosis therapeutic drug is not particularly limited as long as anti-inflammatory adoption and cell proliferation suppression can be obtained by increasing the expression of GDF15, but it is preferably 0.1% or greater, more preferably 1% or greater, and still more preferably 10% or greater.

### <Fibrosis>

Fibrosis as mentioned in the present invention means stiffening accompanying tissue dysfunction due to excessive migration and proliferation of fibroblasts generated in the process of compensating for tissue parenchymal cell shedding or tissue function deterioration that occurs by a stress such as chemical stimulation of a drug and the like, an excessive pressure load, an inflammatory reaction, and the like; and the subsequent synthetic deposition of an intercellular matrix protein. The type or the onset site of the inducing stimulus is not particularly limited. Examples of such tissue fibrotic disease include fibrosis of visceral tissues such as the lung, the bladder, the kidney, the heart, and the liver. Examples of fibrosis targeted by the present invention include tissue fibrotic diseases caused by administration of an agent such as an antitumor agent, an antibiotic, an antibacterial agent, an antiarrhythmic agent, an antiinflammatory agent, an antirheumatic agent, an interferon, and Sho-saiko-to; and tissue fibrotic diseases associated with diseases such as chronic nephritis, interstitial myocarditis, and interstitial cystitis. Specific examples of such fibrosis include pulmonary fibrosis caused by side effects of bleomycin administration, or pulmonary fibrosis occurring during or after interstitial pneumonia; bladder fibrosis or bladder cirrhosis occurring during interstitial cystitis; renal fibrosis caused by genetic abnormalities and the like, and renal failure (renal sclerosis); endocardial fibrosis caused by remodeling after myocardial infarction; liver fibrosis caused by damage to hepatic cells, non-alcoholic steatohepatitis (NASH), and concomitant portal hypertension and cirrhosis; keloids caused by excessive tissue repair, as well as sclerosing peritonitis, prostatic hypertrophy, sclerosis, uterine smooth myoma, retroperitoneal fibrosis, and myeloid fibrosis.

Among the fibrosis therapeutic drugs of the present invention, a fibrosis-suppressing drug ameliorates fibrosis by inhibiting a fibrosis signal in which stellate cells are activated and transformed into myofibroblast-like cells. Therefore, in fibrosis in which stellate cells are activated and turn into myofibroblast-like cells, for example, in the liver, fibrosis proceeds in which the activated stellate cells are transformed into myofibroblast-like cells to produce extracellular matrix, but by using the fibrosis therapeutic drug of the present invention, it is possible to ameliorate the liver fibrosis, improve liver function, and suppress the development of liver cancer. Similarly, it is also possible to use the fibrosis therapeutic drug for pancreatic fibrosis and the like.

### <Method for Treating Fibrosis>

The method for treating fibrosis of the present invention includes binding the above-mentioned therapeutic drug of the present invention to fibrotic tissues or cells. The binding method is not particularly limited, but examples thereof include a method in which a therapeutic agent is directly added to fibrotic tissues or cells, or a formulated fibrosis therapeutic drug is orally or parenterally administered to allow an O-GlcNAcylated protein-like substance to reach an affected area and the vicinity thereof.

### [Examples]

Hereinafter, the present invention will be described in more detail with reference to Examples, but the present invention is not limited to these Examples. In the following, "%" is based on mass unless otherwise specified.

### [Polymerization of O-GlcNAcylated Protein-Like Substance]

### [Polymerization Example 1]

### <Manufacture of GlcNAc-NH₂>

5 g of GlcNAc was dissolved in 50 mL of water and NH₄HCO₃ was added thereto until the solution was saturated. The mixture was stirred in an eggplant flask, incubated at 30°C to 37°C for 4 to 5 days in an open system with an open lid, and NH₄HCO₃ was added as appropriate in a case where the precipitation of NH₄HCO₃ disappeared. The synthesis of GlcNAc-NH₂ was confirmed by thin film chromatography (TLC) with ethyl acetate/acetic acid/methanol/water = 4:3 3:1 (v/v/v/v) and GlcNAc Rf value: 0.71. After 4 to 5 days, water was added to the reaction solution and an evaporation (30°C) was performed to remove excess NH₄HCO₃. Furthermore, this operation was repeated many times until the odor disappeared. After evaporating, freeze-drying was performed.

### <Manufacture of AC-GlcNAc Monomer>

GlcNAc-NH₂ (4.5 mmol: about 1 g) was dissolved in DMSO (10 mL) and 2-carboxyethyl acrylate (4.5 mmol: about 0.65 g) was added thereto. After the dissolution, DMT-MM (6.8 mmol) was added to the mixture and incubation was performed at room temperature for 18 hours. After the incubation, the reactant was added dropwise to 200 to 300 mL of chloroform. A precipitate was obtained, and thus recovered with a Kiriyama funnel. The recovered precipitate was dissolved in methanol, the insoluble matter was removed with a Kiriyama funnel, and the fraction dissolved in methanol was recovered. An evaporation was performed to remove methanol. The solid substance obtained by removing methanol was dissolved in water and then purified by preparative HPLC (water/acetonitrile), acetonitrile was removed therefrom by an evaporator, and the residue was then freeze-dried to obtain an AC-GlcNAc monomer having the following chemical formula.

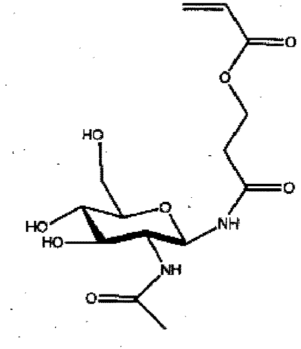

### <Polymerization of AC-GlcNAc Polymer>

(1) 50 mg of an AC-GlcNAc monomer was weighed into a 1 mL microtube and dissolved in 250 µL of dimethyl sulfoxide (DMSO). (2) About 5.26 mg (1/10 molar equivalents of a monomer) of a RAFT agent (2-(dodecylthiocarbonothioylthio)-2-methylpropanoic acid (DTMPA: manufactured by Sigma)) was weighed into a 1 mL microtube, and dissolved in 200 µL. Furthermore, (3) 1 mg (2% with respect to the monomer) of azobisisobutyronitrile (AIBN) was weighed into the microtube, and DMSO was added thereto so that 1 mg of AIBN was dissolved in 50 µL of DMSO. 250 µL of the solution of (1) was mixed with 200 µL of the solution of (2), 50 µL of the solution of (3) was then mixed with the mixture, and after degassing (repeating freeze-and-thaw degassing about three times), incubation was performed at 65°C for about 18 hours to perform polymerization. After the polymerization, the polymer was precipitated with isopropanol, centrifuged, recovered, dissolved in water, and dialyzed (MW100-500) for about 1 day to remove unreacted monomers. After the dialysis, freeze-drying was performed to manufacture an AC-GlcNAc polymer (10-mer, hereinafter referred to as AC-GlcNAc 10).

10 mg of AC-GlcNAc10 was weighed into a 1 mL microtube and dissolved in 100 µL of water. 1 mg of sodium borohydride as a reducing agent was weighed into a 1 mL microtube, and water was added thereto so that 1 mg of the reducing agent was dissolved in 100 µL of water. 100 µL of these solutions were each mixed, the terminals were thiolated, and the solution was then added dropwise to isopropanol (IPA) to recover a precipitate (reactant).

Then, 1 to 3 M sodium acetate was added to water to prepare 100 µL of a solvent adjusted to have a pH of 7 to 8, and the reactant was dissolved therein. Maleimide in the same amount as that of AC-GlcNAc10 was dissolved in 100 µL of a pH-adjusted solvent, and the solution in which the reactant was dissolved was mixed with this solution and left to stand for 1 to 2 hours. This solution was added dropwise to IPA to recover the precipitate, and then the reactant was dissolved in water and freeze-dried to obtain an O-GlcNAcylated protein-like substance.

The mass-average molecular weight and the number-average molecular weight of this product were 4,000 and 3,100, respectively, and each of the average molecular weights was measured under the following conditions using a gel permeation chromatography (GPC) device (product name: LC-9110G NEXT, manufactured by Japan Analytical Industry Co., Ltd.). The column was JAIGEL-GS510 and the eluate was 200 mM sodium nitrate/acetonitrile = 80/20. The flow rate was 1 mL/min, the detector was an RI detector, and the column temperature was 40°C. A standard curve of the molecular weight was obtained with a pullulan.

### [Polymerization Example 2]

An O-GlcNAcylated protein-like substance (referred to as AC-GlcNAc40) was manufactured in the same manner as in Polymerization Example 1, except that a blending ratio of the RAFT agent was set to about 1 mg (1/50 molar equivalents of the monomer) in <Polymerization of AC-GlcNAc Polymer> of Polymerization Example 1.

The mass-average molecular weight and the number-average molecular weight of this product were 14,000 and 6,800, respectively.

### [Polymerization Example 3]

An O-GlcNAcylated protein-like substance (referred to as AC-GlcNAc62) was manufactured in the same manner as in Polymerization Example 1, except that a blending ratio of the RAFT agent was set to about 0.75 mg (1/70 molar equivalents of the monomer) in <Polymerization of AC-GlcNAc Polymer> of Polymerization Example 1.

The mass-average molecular weight and the number-average molecular weight of this product were 21,800 and 12,500, respectively.

### [Polymerization Example 4]

An O-GlcNAcylated protein-like substance (referred to as AC-GlcNAc69) was manufactured in the same manner as in Polymerization Example 1, except that a blending ratio of the RAFT agent was set to about 0.52 mg (1/100 molar equivalents of the monomer) in <Polymerization of AC-GlcNAc Polymer> of Polymerization Example 1.

The mass-average molecular weight and the number-average molecular weight of this product were 24,450 and 12,800, respectively.

### [Polymerization Example 5]

An AC-GlcNAc monomer was manufactured by performing polymerization up to <Manufacture of AC-GlcNAc Monomer> of Polymerization Example 1. The mass-average molecular weight and the number-average molecular weight of this product were both 346.

### [Example 1]

An effect of the O-GlcNAcylated protein-like substance (AC-GlcNAc10) obtained in Polymerization Example 1 on human fibroblasts (NHDF) was confirmed using a DNA microarray.

50 µg/mL of the O-GlcNAcylated protein-like substance obtained in Polymerization Example 1 was added to NHDF, and incubation was performed for 24 hours. After the incubation, mRNA was recovered, and a DNA microarray was performed to confirm a change in the gene expression by the O-GlcNAcylated protein-like substance. The DNA microarray was performed using an Agilent Technologies microarray system. The results are shown in Figs. 1 and 2.

Fig. 1 is a plot of the gene expression levels of NHDF stimulated with an O-GlcNAcylated protein-like substance (vertical axis) and the gene expression levels of unstimulated NHDF (horizontal axis). The left figure shows all the analyzed genes. The right figure plots genes with significantly different expression levels (those with a difference in the expression levels of 1.5 times or greater and those with the difference of 0.66 times or less).

As shown in Fig. 2, the expressions of GDF15 and adiponectin (ADIPOQ) were high by stimulation with the O-GlcNAcylated protein-like substance. From these results, it can be seen that the O-GlcNAcylated protein-like substance increases the expression of GDF15 and adiponectin at the protein level by acting on NHDF.

### [Example 2]

50 µg/mL of the O-GlcNAcylated protein-like substance, the AC-GlcNAc monomer, or N-acetylglucosamine (GlcNAc) prepared in Polymerization Examples 1 to 5 was added to NHDF, and incubation was performed for 24 hours. After the incubation, the expression of NHDF GDF15 was examined by Western blotting. The results are shown in Figs. 3A and 3B. Furthermore, the control was one to which nothing was added.

From these results, it can be seen that the O-GlcNAcylated protein-like substance (AC-GlcNAc10, mass-average molecular weight: 4,000, number-average molecular weight: 3,100) prepared in Polymerization Example 1, the O-GlcNAcylated protein-like substance (AC-GlcNAc40, mass-average molecular weight: 14,000, number-average molecular weight: 6,800) prepared in Polymerization Example 2, the O-GlcNAcylated protein-like substance (AC-GlcNAc62, mass-average molecular weight: 21,800, number-average molecular weight: 12,500) prepared in Polymerization Example 3, and the O-GlcNAcylated protein-like substance (AC-GlcNAc69, mass-average molecular weight: 24,450, number-average molecular weight: 12,800) prepared in Polymerization Example 4 had an increase in the expression of GDF15, but the AC-GlcNAc monomer prepared in Polymerization Example 5 or GlcNAc had almost the same effect as the control.

Furthermore, 10 µg/mL, 50 µg/mL, and 100 µg/mL of the O-GlcNAcylated protein-like substances (AC-GlcNAc10, mass-average molecular weight: 4,000, number-average molecular weight: 3,100) prepared in Polymerization Example 1 were each added to NHDF, and incubation was performed for 24 hours. After the incubation, the expression of NHDF GDF15 was observed by Western blotting. The results are shown in Fig. 3C. Furthermore, the control was one to which nothing was added.

From these results, it can be seen that the O-GlcNAcylated protein-like substance (AC-GlcNAc10, mass-average molecular weight: 4,000, number-average molecular weight: 3,100) prepared in Polymerization Example 1 increased the expression of GDF15 in proportion to the addition amount.

### [Example 3]

From the results of the microarray of Example 1, pathway analysis of differentially expressed genes by the O-GlcNAcylated protein-like substance (AC-GlcNAc10) prepared in Polymerization Example 1 was performed. The results are shown in Fig. 4. All gene clusters having large expression differentiation (shown on the right side in Fig. 1) were input to DAVID Bioinformatics Resources 6.8, which is a pathway analysis software, to perform pathway analysis.

From these results, it can be seen that the blocking of cell proliferation was caused by the O-GlcNAcylated protein-like substance of Polymerization Example 1. In particular, a reduction in genes that progress in the G2 and M phases of the cell cycle was observed.

With regard to this, the interaction of the O-GlcNAcylated protein-like substances with vimentin on the cell surface caused the blocking of a cell cycle through the suppression of a cell division-related function of vimentin. However, in the control to which nothing was added, such a situation did not occur, and thus, the proliferation rate was considered to be higher than that of NHDF on which an O-GlcNAcylated protein-like substance was allowed to act.

### [Example 4]

NHDF was seeded on a 48-well plate, and 50 µg/mL of the O-GlcNAcylated protein-like substance (AC-GlcNAc10) prepared in Polymerization Example 1, the O-GlcNAcylated protein-like substance (AC-GlcNAc40) prepared in Polymerization Example 2, and the AC-GlcNAc monomer and N-acetylglucosamine prepared in Polymerization Example 5 were each added thereto. Thereafter, culture was performed for 6 days, and the cell viability for each day was measured with a Cell Counting Kit-8 (CCK-8) (manufactured by Dojindo Laboratories Co., Ltd). The results are shown in Fig. 5.

From these results, it can be seen that the proliferation of NHDF treated with O-GlcNAcylated protein-like substances (AC-GlcNAc10 and AC-GlcNAc40) is reduced.

### [Example 5]

The anti-inflammatory action of the O-GlcNAcylated protein-like substance was examined as follows.

50 µg/mL of the O-GlcNAcylated protein-like substance (AC-GlcNAc10) prepared in Polymerization Example 1 was added to NHDF, and culture was performed for 24 hours. Lipopolysaccharide (LPS) was added to the NHDF, and a change in the expression of TNFα was observed. In addition, the expression of TNFα in cells at 8 hours after the addition of LPS was examined by Western blotting. The results are shown in Fig. 6A. In addition, the expressions of intracellular GDF15, survivin, and β-actin at 8 hours after the addition of LPS were examined by Western blotting. The results are shown in Fig. 6B. Furthermore, an intracellular ratio of TNFα to β-actin (TNFα/β-actin) at 8 hours after the addition of LPS was examined. The results are shown in Fig. 6C.

As shown in Figs. 6A and 6C, in a case where LPS was allowed to act on NHDF treated with the O-GlcNAcylated protein-like substance prepared in Polymerization Example 1, suppression of an increase in the expression of TNFα by LPS stimulation was observed. In addition, as shown in Fig. 6B, in NHDF treated with the O-GlcNAcylated protein-like substance prepared in Polymerization Example 1, an increase in the expression of GDF15 and suppression of the expression of survivin were observed. GDF15 is a type of TGFβ superfamily and is known to inhibit the transcriptional activity of NF-κB. Therefore, it is presumed that due to the increase in the expression of GDF15 by the treatment of NHDF with the O-GlcNAcylated protein-like substance, the activation of NF-κB was suppressed and the expression of TNFα was decreased.

Next, the amount of TNF-α secreted into the medium at 24 hours after the addition of LPS to NHDF was measured by ELISA. NHDF was subjected to a culture medium exchange with DMEM (serum-free), and 50 µg/mL of the O-GlcNAcylated protein-like substance (AC-GlcNAc10) was added to the medium. 24 hours after the addition of AC-GlcNAc10, 5 µg/mL of LPS was added thereto, 24 hours later, the culture supernatant was recovered, and TNF-α included in the culture supernatant was measured by ELISA. At this time, TNF-α included in each of the DMEM culture supernatant (control) of NHDF, the culture supernatant (AC-GlcNAc10) at 48 hours after the addition of 50 µg/mL of AC-GlcMAc10 to NHDF, and the culture supernatant at 24 hours after the addition of 5 µg/mL of LPS to NHDF was also measured by ELISA. The results are shown in Fig. 7. As shown in Fig. 7, there was no change in the amount of TNFα secreted in those without the addition of the O-GlcNAcylated protein-like substance (control) and those with the addition of the O-GlcNAcylated protein-like substance, but an increase in the amount of TNFα secreted was observed in those with the addition of LPS to the control, and suppression of an increase in the amount of TNFα secreted by LPS was observed in those with the addition of LPS to those to which the O-GlcNAcylated protein-like substance was added.

### [Example 6]

It was examined as follows whether stimulation by the addition of an O-GlcNAcylated protein-like substance to NHDF has an effect on the cell migration ability.

Using AC-GlcNAc (10-mer) as an O-GlcNAcylated protein-like substance, 250 µg/mLAC-GlcNAc (10-mer) was mixed with an agarose gel to prepare an O-GlcNAcylated protein-like substance-containing agarose gel. In addition, a PBS-containing agarose gel was prepared using PBS instead of the O-GlcNAcylated protein-like substance. A glass plate was placed in a petri dish, each agarose gel was added dropwise onto the glass plate, NHDF was further seeded on the entire glass plate, and incubation was performed for one day.

Fig. 8 shows the results of observing the state of NHDF at this time with a phase-contrast microscope; and observing the state of invasion into cells on the bottom surface of the agarose gel. From the results shown in Fig. 8, it can be seen that the O-GlcNAcylated protein-like substance imparts chemotaxis of NHDF from the fact that NHDF enters under the agarose gel containing the O-GlcNAcylated protein-like substance (AC-GlcNAc10) of Polymerization Example 1.

Next, agarose gels having blending amounts of 50 µg/mL, 100 µg/mL, and 250 µg/mL of the O-GlcNAcylated protein-like substance were prepared, and the chemotaxis of NHDF was evaluated in the same manner as above. The results are shown in Fig. 9. From the results shown in Fig. 9, the chemotaxis of NHDF varied depending on the concentration of the O-GlcNAcylated protein-like substance. Specifically, in a case where the concentration of the O-GlcNAcylated protein-like substance was 250 µg/mL, the distance over which the cells entered the gel was 750 µm and the number of cells that entered the gel was approximately 60. In a case where the concentration of the O-GlcNAcylated protein-like substance was 100 µg/mL, the above-mentioned distance was 450 µm and the above-mentioned number of cells was approximately 12. In a case where the concentration of the O-GlcNAcylated protein-like substance was 50 µg/mL, the above-mentioned distance was 280 µm and the above-mentioned number of cells was approximately 10, and in the agarose gel containing PBS, both of the above-mentioned distance and the above-mentioned number of cells were 0. From this, it can be seen that the chemotaxis of NHDF depends on the concentration of the O-GlcNAcylated protein-like substance.

Next, the chemotaxis of NHDF by an O-GlcNAcylated protein-like substance was evaluated. The materials used were the O-GlcNAcylated protein-like substance prepared in Polymerization Example 1, the AC-GlcNAc monomer prepared in Polymerization Example 5, and N-acetylglucosamine (GlcNAc) and PBS used in Example 2, and the chemotaxis of NHDF was evaluated in the same manner as above. The results are shown in Fig. 10. As shown in Fig. 10, the cells invaded under the agarose gel only with the O-GlcNAcylated protein-like substance of Polymerization Example 1, which is a polymer, and the distance over which NHDF entered was 420 µm and the number of NHDFs that entered was 29. From this, it can be seen that since the O-GlcNAcylated protein-like substance is a polymer, NHDF is allowed to act thereon.

Next, it was evaluated by the same method as above whether or not there was a difference in the chemotaxis of NHDF due to the difference in the degree of polymerization of the O-GlcNAcylated protein-like substance. In the evaluation, as the O-GlcNAcylated protein-like substance, the O-GlcNAcylated protein-like substance (AC-GlcNAc10) prepared in Polymerization Example 1 and the O-GlcNAcylated protein-like substance (AC-GlcNAc40) prepared in Polymerization Example 2 were used. The results are shown in Fig. 11. From the results shown in Fig. 11, the cells invaded under the agarose gel with any of the O-GlcNAcylated protein-like substances, but the distance over which NHDF entered was 780 µm with the O-GlcNAcylated protein-like substance prepared in Polymerization Example 1, and the distance over which NHDF entered was 600 µm with the O-GlcNAcylated protein-like substance prepared in Polymerization Example 2. Further, the number of NHDFs that entered the gel was 60 with the O-GlcNAcylated protein-like substance prepared in Polymerization Example 1, and the number of NHDFs that entered the gel was 50 with the O-GlcNAcylated protein-like substance prepared in Polymerization Example 2.

In addition, two petri dishes in which a gel containing the O-GlcNAcylated protein-like substance (AC-GlcNAc10)prepared in Polymerization Example 1 was added dropwise onto an agarose gel were prepared, and 10 µg/mL of the O-GlcNAcylated protein-like substance (AC-GlcNAc10) prepared in Polymerization Example 1 was incorporated into a medium in one of the petri dishes to evaluate the chemotaxis of NHDF. The results are shown in Fig. 12. From the results shown in Fig. 12, it can be seen that in a case where a medium contains an O-GlcNAcylated protein-like substance, invasion of NHDF under the agarose gel is reduced. Specifically, the distance over which NHDF entered in a case of using a medium including the O-GlcNAcylated protein-like substance was 1,000 µm, but the distance over which NHDF entered in a case of using a medium including no O-GlcNAcylated protein-like substance was 1,250 µm. The number of NHDFs that entered in a case of using a medium including the O-GlcNAcylated protein-like substance was 80, and the number of NHDFs that entered in a case of using a medium including no O-GlcNAcylated protein-like substance was 140. Incidentally, in the agarose gel containing PBS, NHDF did not invade the gel.

Furthermore, the migration stimulation of NHDF by the O-GlcNAcylated protein-like substance (AC-GlcNAc10) prepared in Polymerization Example 1 was examined by detecting the phosphorylation of vimentin. From the fact that the O-GlcNAcylated protein-like substance binds to vimentin appearing on the cell surface, the signal transduction to vimentin by the addition of the O-GlcNAcylated protein-like substance was examined by the phosphorylation of the 38^{th} serine in the amino acid sequence of vimentin. It was known that cell migration is stimulated by the phosphorylation of the 38^{th} serine of vimentin. Therefore, the phosphorylation of vimentin was detected by Western blotting with an anti-phosphorylated vimentin serine-38 antibody at 10 minutes, 30 minutes, 60 minutes, and 120 minutes after the addition of the O-GlcNAcylated protein-like substance to NHDF. The results are shown in Fig. 13. As shown in Fig. 13, the phosphorylation of vimentin was observed 10 minutes after the addition of the O-GlcNAcylated protein-like substance, and a reduction in the phosphorylation was observed 120 minutes after the addition. From this, it was clarified that the O-GlcNAcylated protein-like substance binds to vimentin on the cell surface of NHDF and stimulates the expression of GDF15 or the cell migration.

From the results above, it was confirmed that NHDF treated with an O-GlcNAcylated protein-like substance has a high cell migration ability. In addition, as shown in Fig. 13, from the fact that the phosphorylation of vimentin associated with the stimulation of cell-surface vimentin by O-GlcNAcylated protein-like substance (AC-GlcNAc10) was also observed, it is considered that the cell migration ability was stimulated by the polymerization of vimentin and the accompanying signal transduction.

### [Example 7]

50 µg/mL of the O-GlcNAcylated protein-like substance (AC-GlcNAc10) prepared in Polymerization Example 1 was added to osteoarthritis-derived synovial fibroblasts and rheumatoid arthritis-derived synovial fibroblasts, and culture was performed for 24 hours. The expression of GDF15 in the cells at this time was examined by Western blotting. The results are shown in Fig. 14A. In addition, 10 ng/mL of TNFα was added to the osteoarthritis-derived synovial fibroblasts and the rheumatoid arthritis-derived synovial fibroblasts, and the expression of RANKL, survivin, and β-actin at 6 hours after the addition of TNFα was examined by Western blotting. The results are shown in Fig. 14B.

As shown in Fig. 14A, in any of the osteoarthritis-derived synovial fibroblasts and rheumatoid arthritis-derived synovial fibroblasts treated with the O-GlcNAcylated protein-like substance prepared in Polymerization Example 1, an increase in the expression of GDF15 was observed, and as shown in Fig. 14B, suppression of an increase in the expression of RANKL and survivin by TNFα stimulation was observed.

In the diseased area of rheumatoid arthritis, which is a chronic inflammatory disease, synovial fibroblasts grow to express RANKL and activate osteoclasts, and the exacerbation of the disease progresses. In addition, RANKL is secreted from the synovial fibroblasts stimulated by TNFα or interleukin-17 (hereinafter referred to as IL-17) during chronic inflammation to differentiate osteoclast precursor cells into osteoclasts, and the osteoclasts cause bone destruction which causes bone deformities in rheumatoid arthritis. As shown in Figs. 14A and 14B, in a case where an O-GlcNAcylated protein-like substance (AC-GlcNAc10) was allowed to act on rheumatoid arthritis-derived synovial fibroblasts and TNFα was allowed to act thereon, an increase in the expression of GDF15 and suppression of an increase in the expression of RANKL and survivin were observed. From this, it is presumed that bone fibroblasts and rheumatoid arthritis-derived synovial fibroblasts on which an O-GlcNAcylated protein-like substance (AC-GlcNAc10)was allowed to act suppressed an increase in the expression of RANKL, and as a result, the differentiation into osteoclasts can be suppressed and the onset of rheumatoid arthritis can be suppressed.

Furthermore, survivin is a molecule that suppresses cell death and is known to be involved in the activation of synovial fibroblasts, and from the fact that suppression of an increase in the expression of RANKL or survivin in the cells due to the O-GlcNAcylated protein-like substance (AC-GlcNAc10) was observed, it is presumed that the O-GlcNAcylated protein-like substance has a therapeutic effect on a rheumatoid arthritis disease or an effect of suppressing drug resistance to various drugs including a cancer therapeutic drug.

From the results of Examples 1 to 6, it was found that GDF15 production, an anti-inflammatory action or proliferation suppression, and an increase in cell migration ability were obtained by the stimulation of cell-surface vimentin by the O-GlcNAcylated protein-like substance in myofibroblasts (NHDF). From this, it is presumed that the O-GlcNAcylated protein-like substance has a therapeutic effect on fibrosis.

Furthermore, from the results of Example 7, it was found that periosteal fibroblasts and rheumatoid arthritis-derived synovial fibroblasts on which the O-GlcNAcylated protein-like substance (AC-GlcNAc10) was allowed to act suppressed the expression of RANKL and survivin even in a case where TNFα was allowed to act. From this, it is presumed that the O-GlcNAcylated protein-like substance has a therapeutic effect on rheumatoid arthritis and an inhibitory effect on drug resistance.

## Claims

1. An O-GlcNAcylated protein-like substance comprising an N-acetylglucosamine unit and at least one unit selected from the group consisting of a carboxy group-containing radically polymerizable unit, a styrene unit, a polyethylenimine unit, a poly-L-lysine unit, and a biotin unit.

2. The O-GlcNAcylated protein-like substance according to Claim 1,
wherein the carboxy group-containing radically polymerizable unit is at least one selected from the group consisting of acrylic acid, methacrylic acid, itaconic acid, maleic acid, crotonic acid, 2-carboxyethyl acrylate, and 2-carboxyethyl methacrylate.

3. The O-GlcNAcylated protein-like substance according to Claim 1 or 2,
wherein the O-GlcNAcylated protein-like substance has no alkyl chain.

4. The O-GlcNAcylated protein-like substance according to any one of Claims 1 to 3,
wherein the O-GlcNAcylated protein-like substance is a compound represented by Chemical Formula (1) or (2), 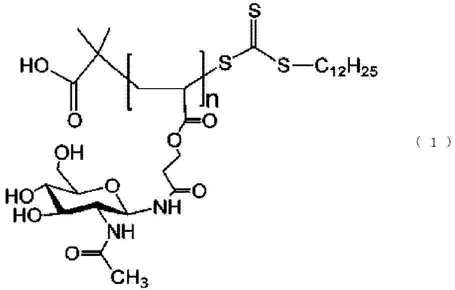 in Formula (1), n is an integer of 3 to 15, 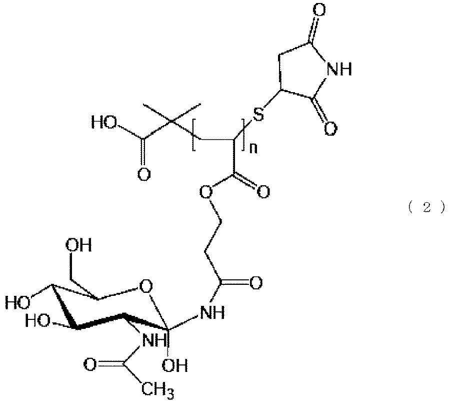 in Formula (2), n is an integer of 3 to 15.

5. A fibrosis therapeutic drug comprising the O-GlcNAcylated protein-like substance according to any one of Claims 1 to 4 as an active ingredient.
